# EUROPÄISCHE PATENTSCHRIFT

(11) **EP 1 600 768 B1**
(45) Veröffentlichungstag und Bekanntmachung des Hinweises auf die Patenterteilung: **13.03.2013**
(21) Anmeldenummer: 05405355.8
(22) Anmeldetag: 20.05.2005
(51) Int. Cl.: G01N 27/49, G01N 33/00

(54) **Ammoniaksensor**
Ammonia sensor
Capteur d'ammoniac

(30) Priorität: 27.05.2004 CH 9042004
(43) Veröffentlichungstag der Anmeldung: 30.11.2005
(73) Patentinhaber: Membrapor AG, 8304 Wallisellen (CH)
(72) Erfinder: Huggenberger, Christian, 8048 Zürich (CH); Nezel, Tomas, 5430 Wettingen (CH)
(74) Vertreter: Cremer & Cremer

(56) Entgegenhaltungen:
- EP-A- 0 496 527
- EP-A- 0 693 180
- US-A- 3 776 832
- US-A- 4 227 984
- US-A- 5 344 546
- US-A- 5 910 239
- TOMAS NEZEL: "Gassensoren" SKRIPT ZUR VORLESUNG GASSENSOREN, [Online] November 2000 (2000-11), Seiten 1-11, XP002334987 ETH Zürich Gefunden im Internet: URL:www.chemsens.ethz.ch/~tomas/pdf_files/ skript.pdf> [gefunden am 2005-07-06]
- "Chemical Sensing with Solid State Devices Passage ED - Madou M; Morrison S", 1 January 1989 (1989-01-01), CHEMICAL SENSING WITH SOLID STATE DEVICES, ACADEMIC PRESS, US, PAGE(S) 1 - 6, XP007919455, ISBN: 0-12-464965-3
- STEFAN VAIHINGER: "Mehrkomponentenanalyse durch zeitabhängige Signale chemischer Gassensoren", 19920101; 1992 - 1992, 1 January 1992 (1992-01-01), pages 1-3, XP007919459, [retrieved on 1992-01-01]

## Beschreibung

Die Erfindung betrifft einen Ammoniaksensor gemäss dem Oberbegriff des Patentanspruchs 1.

Seit längerer Zeit sind am Markt amperometrische Ammoniak-Gassensoren bekannt, deren Elektrolyt ein Co(II)-Salz (Reagenz für NH₃) sowie Ethylenglykol als hygroskopischen Zusatz enthält. Dieser hält die Wasserbalance des Elektrolyten mit der Umgebungsfeuchte (üblicherweise zwischen 15 % und 90 % R.F.) aufrecht. Die bekannte Messzelle, die ohne Biasspannung auskommt, ist eine Drei-Elektroden-Messzelle mit einer Arbeitselektrode, Gegenelektrode und einer Referenzelektrode und wird mit einem Potentiostat betrieben. Die Arbeitselektroden und Referenzelektroden sind aus identischen Materialen (Gold-Grafit Doppelschichten) hergestellt. Solche Messzellen sind in EP 0693180 B1 beschrieben. Diese Sensoren weisen einen zum idealen Nullpunkt stark asymmetrisch verschobenen, negativen, zum Ammoniakgas-Signalstrom stets entgegengesetzten Basislinienstrom auf (typischerweise -500 nA bei Raumtemperatur, entsprechend - 5 ppm Ammoniak). Der Basislinienstrom ist zudem unüblich stark temperaturabhängig und erreicht den Endwert stark verzögert. Der verschobene Basislinienstrom beeinträchtigt eine genaue Detektion von Ammoniakgas im Bereich des TLV-Wertes oder darunter, besonders bei Umgebungstemperaturen oberhalb von 25 °C.

Allgemein weisen elektrochemische Dreielektroden-Gassensoren wesentlich geringere Basislinienströme auf. Die potentiometrisch (das heisst stromlos) betriebene Referenzelektrode, die wie die Arbeits- und Gegenelektrode als Gasdiffusionselektrode konzipiert ist, generiert im Kontakt mit Elektrolyt/Luft ein Referenzpotenzial, wobei die Luft im rückseitigen, porösen PTFE-Träger sowie gelöst im Elektrolyten zur Verfügung steht. Den Regeln der Kunst entsprechend ist die Referenzelektrode im Elektrolyt eingebettet und gegen das Messgas möglichst abgeschirmt, damit dieses die Referenzelektrode nicht irritieren kann. Die Referenzelektrode liefert einen Bezugspunkt für den Potentiostat, damit dieser die Arbeitselektrode stets auf einem konstanten Potenzial relativ zur Referenzelektrode halten kann. Falls Referenz- und Arbeitselektroden auf demselben Potenzial gehalten werden, ist in reiner Umgebungsluft der Basislinienstrom im Idealfall praktisch Null.

Die starke Basislinienverschiebung beim oben beschriebenen Ammoniaksensor kann dem Ethylenglykol zugeschrieben werden. Versuche, Ethylenglykol durch andere, bei elektrochemischen Sensoren üblicherweise verwendete hygroskopische Bestandteile des Elektrolyten zu ersetzen, wie durch Säuren, Basen, hygroskopische Salze, die eine Wasserbalance des Elektrolyten mit der Umgebungsfeuchte (üblich zwischen 15% und 90 % R.F) aufrechterhalten, brachten negative Resultate, da das bezüglich dem Messgas NH3 aktive Reagenz Co²⁺ in Gegenwart dieser Substanzen auf verschiedene Weise inaktiviert wird.

Eine Korrektur des Basislinienstromes auf elektronischem Wege erweist sich als zu ungenau, da dieser einer Temperaturänderung stark verzögert folgt und von Sensor zu Sensor stark streut.

Der Basislinienstrom könnte hingegen mit Hilfe einer zusätzlichen Hilfselektrode reduziert werden, mit einer 4-Elektroden-Anordnung, die in EP 0126623 (B2) beschrieben wird und einer Basislinienkorrektur, die in EP 0127387 (A2) erwähnt ist. Ein von der Firma City Technology Ltd hergestellter Ammoniaksensor benützt dieses Prinzip, um den grossen, stark positiven und stark temperaturabhängigen Basislinienstrom, der durch eine angelegte Biasspannung verursacht wird, teilweise zu kompensieren. Eine Hilfselektrode, auf die das Messgas keinen Zutritt hat, erzeugt einen zweiten, praktisch gleichgrossen und gleichermassen temperaturabhängigen Basislinienstrom. Mit dem Basislinienstrom der Hilfselektrode kann somit der Basislinienstrom der Messzelle elektronisch korrigiert werden. Nachteilig an dieser Methode ist allerdings, dass eine spezielle Auswerteelektronik mit zwei Schaltkreisen benötigt wird, und der Sensor nicht in bestehenden Auswertegeräten für Dreielektroden-Sensoren eingesetzt werden kann.

Ein weiteres nachteiliges Verhalten des oben beschriebenen, Co²⁺ haltigen Ammoniaksensors ist die Empfindlichkeit gegenüber abrupten Luftfeuchtigkeitsänderungen. Beim schnellen Feuchtigkeitsanstieg der Umgebungsluft, was beispielsweise beim Reinigen von Räumen mit wässrigen Reinigungsmitteln auftreten kann, reagiert der Sensor mit einem transienten Signal, das über Minuten eine Ammoniakkonzentration von bis zu 20 ppm vorspiegelt, was einen Fehlalarm auslösen kann.

Die Erfindung stellt sich daher folgende Aufgabe:
1. Der in EP 0693180 B1 beschriebene, Co²⁺ enthaltende Ammoniaksensor soll derart verbessert werden, dass der Basislinienstrom und dessen Temperaturabhängigkeit signifikant reduziert wird.
2. Im weiteren soll der Sensor derart verbessert werden, dass ein Fehlalarm, bedingt durch eine schlagartige Erhöhung der relativen Luftfeuchtigkeit, ausgeschlossen wird.

Die Aufgabe Teil a) wird mit den erfindungsgemässen Merkmalen nach dem kennzeichnenden Teil des Patentanspruches 1 gelöst. Vorteilhafte Weiterbildungen sind Gegenstand der Unteransprüche. Die Aufgabe Teil b) wird gemäss Anspruch 5 gelöst.

Der vorgeschlagene Ammoniaksensor, dessen Elektrolyt Co²⁺-Ionen und einen mit Luftsauerstoff oxidierbaren hygroskopischen Zusatz wie zum Beispiel Ethylenglykol oder Glyzerin enthält, besitzt einen Zutritt für Luftsauerstoff zur Referenzelektrode, durch den ein Defizit von Sauerstoff an der Referenzelektrode permanent ausgeglichen werden kann, so dass die Referenzelektrode praktisch dieselbe Sauerstoffkonzentration und dieselbe Elektrolytumgebung wie die Arbeitselektrode wahrnimmt und in Verbindung mit dem Potentiostat ein entsprechendes Referenzpotenzial festlegt, so dass der Basislinienstrom nahe beim idealen Nullpunkt liegt.

Dieser Zutritt von Luftsauerstoff erfolgt über einen Gasphasenpfad, der über eine Eintrittsöffnung am Sensorgehäuse und Diffusionskanäle innerhalb des Sensorgehäuses verläuft, so dass der Luftsauerstoff weder durch den Elektrolyten, noch durch einen festen Kunststoff hindurch diffundieren muss. Vorzugsweise weist dieser Gasphasenpfad mindestens eine Diffusionsbarriere, die den Gas-Zutritt zur Referenzelektrode steuert, so dass ein Defizit von Luftsauerstoff jederzeit genügend ausgeglichen wird, andererseits aber der Zutritt von Messgas an die Referenzelektrode, ebenso der Zutritt allfälliger interferierender Bestandteile des Messgases, gegen die der Sensor eine Querempfindlichkeit aufweist, erschwert wird. Vorzugsweise wird der Zutritt des Messgases zur Referenzelektrode gänzlich versperrt. Dies kann beispielsweise durch das Einfügen einer festen Ringscheibe aus Kunststoff wie beispielsweise PVDC (Polyvinylidenchlorid), PC (Polycarbonat), PE (Polyethylen) oder PP (Polyethylen) erreicht werden. Vorzugsweise ist der Katalysator der Messelektrode derart aktiv, dass das zur Messelektrode diffundierende Ammoniakgas praktisch vollständig wegreagiert, was den Zutritt des Messgases zur Referenzelektrode weiter einschränkt.

Der Effekt des vorgeschlagenen Zutritts für Luftsauerstoff zur Referenzelektrode ist verblüffend. Der Basislinienstrom und dessen Temperaturabhängigkeit reduziert sich drastisch, sobald Sauerstoff zur Referenzelektrode geleitet werden kann. Offenbar wird an der Referenzelektrode laufend Sauerstoff verbraucht, was folgern lässt, dass dort Ethylenglykol, Glyzerin, beziehungsweise jeder mit Luftsauerstoff oxidierbare Elektrolyt-Zusatz am aktiven Elektrodenkatalysator der Referenzelektrode oxidiert wird, obwohl die Referenzelektrode stromlos betrieben wird und normalerweise an dieser kein Stoffumsatz, also kein Sauerstoffverbrauch erfolgt.

Der vorgeschlagene Zutritt für Luftsauerstoff zur Referenzelektrode eröffnet noch weitere überraschende Vorteile: Bei vielen elektrochemischen Gas-Sensoren, werden Edelmetall-Katalysatoren der Platinmetallgruppe wie Pt, Ru, Ir eingesetzt, da diese besonders gut und teilweise selektiv auf entsprechende Messgase ansprechen. Beim in EP 0693180 B1 beschriebenen Ammoniaksensor sind solche Elektroden jedoch nicht vorteilhaft, da Platinmetalle mit hygroskopischen Zusätzen wie Ethylenglykol oder Glyzerin und Alkoholen im Allgemeinen stark reagieren können, was den Basislinienstrom noch stärker ansteigen lässt. Beim vorgeschlagenen Ammoniaksensor werden jedoch Platinmetallkatalysatoren wie beispielsweise Ruthenium bevorzugt eingesetzt, da diese kein transientes Signal bezüglich einer Luftfeuchtigkeitsänderung erzeugen, wobei der Basislinienstrom und dessen Temperaturabhängigkeit weiterhin durch den Sauerstoffzutritt zur Referenzelektrode effektiv minimiert und vernachlässigbar klein ist. Als zusätzlicher überraschender Effekt vergrössert sich die Signalintensität bezüglich Ammoniak bei Verwendung von Ruthenium als Elektrodenkatalysator.

Die Erfindung ist unter anderem in den Zeichnungen erläutert. Es zeigen:
- Fig. 1: Schematische Darstellung eines erfindungsgemässen Ammoniaksensors
- Fig. 2: Längsschnitt durch Gehäuse und Elektrodenstapel eines Ammoniaksensors gemäss einer ersten Ausbildungsart
- Fig. 3: Temperaturabhängigkeit des Basislinienstroms

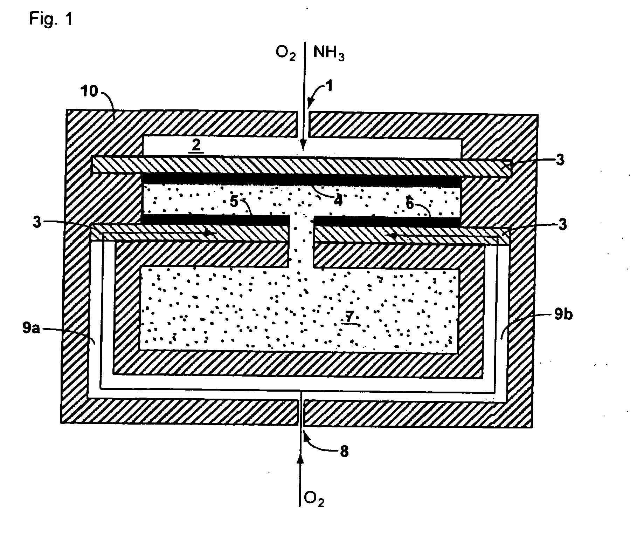

Fig. 1 zeigt schematisch den erfindungsgemässen Ammoniaksensor. Die Messzelle besteht aus einem Gehäuse (10) mit einer Eintrittsöffnung (1) für das Messgas (Ammoniak in Luft), einer rückseitigen Eintrittsöffnung (8) für Luftsauerstoff, drei Gasdiffusionselektroden (Arbeitselektrode (3,4), Referenzelektrode (3,5), Gegenelektrode (3,6)), sowie dem Elektrolyten (7). Über drei Kontaktierungsdrähte (nicht eingezeichnet), welche die Elektrodenkatalysatoren (4,5,6) kontaktieren, ist die Messzelle mit einem Potentiostat (nicht eingezeichnet) verbunden. Der Elektrolyt (7) enthält Co²⁺ und einen mit Luftsauerstoff oxidierbaren hygroskopischen Zusatz, wie beispielsweise Ethylenglykol oder Glyzerin. Sauerstoff der äusseren Umgebungsluft gelangt durch die Eintrittsöffnung (8), die eine Gasdiffusionsbarriere bildet, ins Innere des Gehäuses (10) und diffundiert durch einen Luftspalt (8a) und durch den porösen, Elektrolyt abweisenden Träger (3) zur Katalysatorschicht (5) der Referenzelektrode (3,5), um ein Defizit von Luftsauerstoff auszugleichen zu können. Luftspalt (8a) und Träger (3) können ebenfalls als Diffusionsbarrieren wirken. Auf der Gegenseite diffundiert das Messgas Ammoniak, gesteuert durch die Eintrittsöffnung (1), die eine Gasdiffusionsbarriere bildet, in die innere Gaskammer (2) und zur Arbeitselektrode (3,4), wo es wegreagiert. Als Gegenreaktion wird an der Gegenelektrode (3,6) Sauerstoff reduziert, der ebenfalls über die rückseitige Eintrittsöffnung (8) und über den Luftspalt (8a) nachgeliefert wird. Der gemessene Messzellenstrom ist in guter Näherung linear zur Ammoniakgas-Konzentration. In Abwesenheit von Ammoniak an der Eintrittöffnung (1) wird ein Basislinienstrom gemessen, der nahe beim Nullpunkt liegt. Selbstverständlich kann der Ammoniaksensor mit weiteren Hilfselektroden wie Scavenging-Elektroden ausgestattet werden, die beispielsweise interferierende Bestandteile des Messgases unterdrücken. Der beschriebene Sensor eignet sich für die Detektion von Ammoniak, Aminen, Hydrazin, Silan, Arsin, Phosphin und deren Derivaten in Luft oder in flüssigen Messproben.

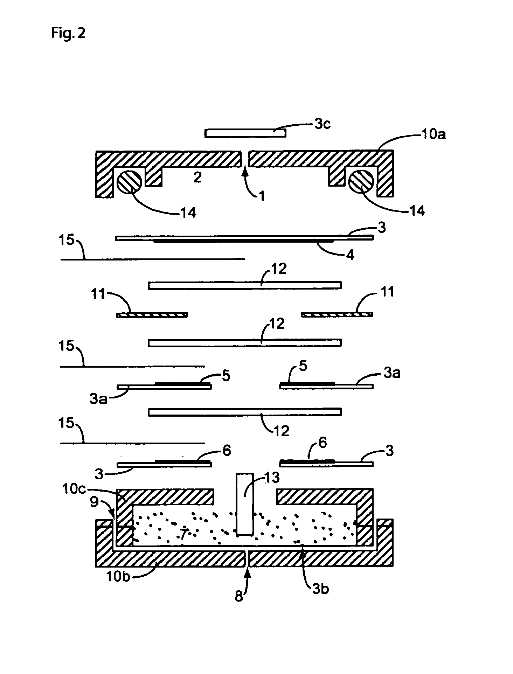

In einer ersten Ausbildungsart gemäss Fig. 2 besteht der Elektrodenstapel (16) eines Ammoniaksensors in Kompaktgrösse aus je einer ringförmigen Gegenelektrode (3,6) und Referenzelektrode (3,5) und einer scheibenförmigen Arbeitselektrode (3,4) mit dazwischenliegenden, scheibenförmigen Glasfaser-Separatoren (12). Zwischen Arbeitselektrode (3,4) und Referenzelektrode (3,5) liegt eine PVDC-Ringscheibe (11), die eine Gas-Sperre bildet. Die Elektrolytversorgung der Elektroden-Katalysatorschichten (4,5,6) erfolgt über die Glasfaser-Separatoren (12) und über einen Glasfaser-Docht (13), der in ein teilweise mit Elektrolyt (7) gefülltes Reservoir des Gehäusekörpers (10c) eintaucht. Am Boden ist der Gehäusekörper (10c) mit einer porösen PTFE-Membran (3b) dichtend verschlossen, wobei dieser vom Gehäuseteil (10b) umfangen wird. Gehäuseteil (10b) besitzt ein kleines, rückseitiges Loch (8), durch das Luft transversal durch Membran (3b) und durch einen von den Gehäuseteilen (10b), (10c) und (10a) gebildeten Luftspalt (9) zur Referenzelektrode (3,5) gelangen kann. Der gasseitige Deckel (10a), der über der Eintrittsöffnung (1) mit einer PTFE-Membran (3c) abgeschlossen ist, wird mit dem unteren Gehäuseteil (10b) verschweisst, so dass der NBR-O-Ring (14) stark komprimiert wird und dadurch die Elektrodenmembranen (3) und die PVDC-Ringscheibe (11) dichtend zusammenpresst werden, so dass der Elektrolyt im Innern des Elektrodenstapels (16) verbleibt. Die Elektroden werden durch die Kontaktdrähte (15) kontaktiert. Die Elektroden sind Gasdiffusionselektroden mit einem Ruthenium-Katalysator. Eine Paste mit Ruthenium-Black und feinen PTFE-Teilchen wird auf eine poröse, 0,2 mm dicke PTFE-Mernbram aufgetragen und getrocknet. Der Aussendurchmesser der PTFE-Membranen beträgt für alle Elektroden 25 mm. Der Aussendurchmesser der zentrisch aufgebrachten Katalysatorschichten beträgt 18 mm. Die Ringelektroden (Referenz-, Gegenelektroden) erhält man aus diesen Elektroden durch Ausstanzen des inneren Teils mit einem Durchmesser von 7 mm. Die PVDC-Ringscheibe (11) wird aus einer 0,05 mm dicken PVDC-Folie mit Innendurchmesser 17 mm und Aussendurchmesser 25 mm ausgestanzt. Die Glasfaser-Separatoren (12) sind Glasfaserscheiben mit einem Durchmesser von 20 mm. Der Elektrolyt (7) enthält eine Konzentration von 0,05 molal CoSO₄ 7H₂O in einem Gemisch von (30 Mass.-%) Wasser und (70 Mass.-%) Ethylenglykol. Die gasseitige Eintrittsöffnung (1) besteht aus 4 Kapillaren mit Durchmessern von 1,3 mm und einer Länge von je 1,8 mm. Das rückseitige Loch (8) hat einen Durchmesser von 0,5 mm. Der mit einem Potentiostat verbundene Ammoniaksensor hat bei Raumtemperatur eine Basislinie von -40 nA. Beim Begasen mit Ammoniak steigt der Sensor rasch auf ein Signal-Plateau. Die Empfindlichkeit bezüglich Ammoniakgas beträgt 105 nA/ppm. Schlagartige Änderungen der Luftfeuchtigkeit erzeugen keine messbaren Effekte.

In einer zweiten Ausbildungsart gemäss Fig. 2 wird ein Ammoniaksensor in

Miniaturgrösse aufgebaut, wobei der Elektrodenstapel (16) einen Aussendurchmesser von 15 mm aufweist. Die Ruthenium-Elektroden werden auf gleiche Weise wie in der ersten Ausbildungsart hergestellt. Der äussere Durchmesser der Katalysatorschichten (4,5,6) beträgt 11 mm. Die beiden Ringelektroden (Referenzelektrode (3,5), bzw. Gegenelektrode (3,6)) haben einen Innendurchmesser von 4,5 mm. Die PVDC-Ringscheibe (11) hat gegenüber den Elektrodenträgern (3) einen etwas geringeren Aussendurchmesser von 14,9 mm und einen Innendurchmesser von 9 mm. Die Glasfaser-Separatoren (12) haben einen Durchmesser von 11 mm. Der Elektrolyt enthält eine Konzentration von 0,05 molal CoSO₄ 7 H₂O in einem Gemisch von (40 Mass.-%) Wasser und (60 Mass.-%) Glyzerin. Die gasseitige Eintrittsöffnung (1) besteht aus 4 Kapillaren mit Durchmessern von 1,5 mm und einer Länge von 1,5 mm. Im Unterschied zur ersten Ausführungsart besitzt dieser Miniatur-Sensor hinten keinen rückseitigen Luftzutritt (8). Der Luftzutritt zur Referenzelektrode (3,5) und Gegenelektrode (3,6) erfolgt durch die Messgas-Eintrittsöffnung (1). Luftsauerstoff kann transversal durch den Träger (3) der Arbeitselektrode (3,4) und über einen kleinen Spalt rings um die PVDC-Ringscheibe (11) wiederum transversal zur Referenzelektrode (3,5) und Gegenelektrode (3,6) diffundieren. Wird der Sensor mit Ammoniakgas exponiert, diffundiert Ammoniak, gesteuert durch die Diffusionsbarriere (1) zur Arbeitselektrode (3,4), wo es am aktiven Ruthenium-Katalysator (4) laufend und praktisch vollständig wegreagiert. Restliche Ammoniakmoleküle, die vor allem bei höheren Ammoniakkonzentration auftreten können, werden durch die innere, transversal durch die Elektroden-Träger (3) gebildete Diffusionsbarriere und die PVDC-Gassperre (11) vom Zutritt auf die Referenzelektrode (3,5) abgeschirmt. Der mit einem Potentiostat verbundene Miniatursensor hat bei Raumtemperatur eine Basislinie von 15 nA mit einer Empfindlichkeit von 81 nA/ppm bezüglich Ammoniak. Die Basislinie reagiert nicht auf Luftfeuchtigkeitsänderungen.

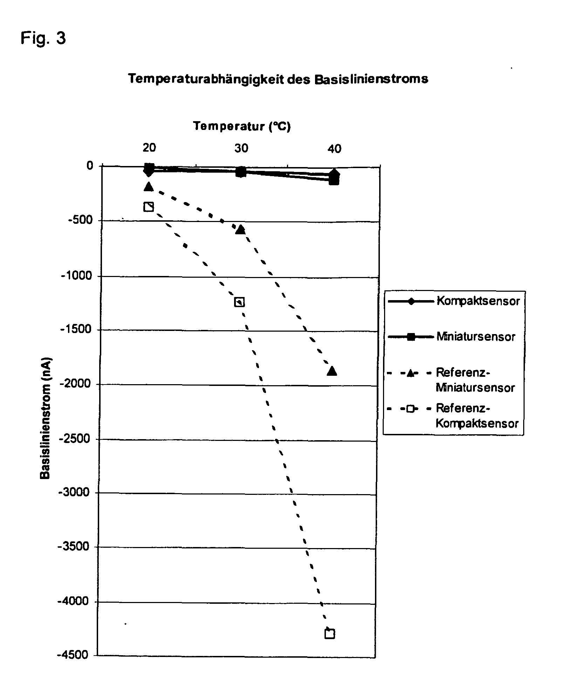

In Fig. 3 ist die Temperaturabhängigkeit des Basislinienstroms der beiden Ausführungsbeispiele (Kompaktsensor bzw. Miniatursensor) wiedergegeben. Im Gegensatz zu einem gleichartigen Referenz-Kompaktsensor und einem gleichartigen Referenz-Miniatursensor, die auf analoge Weise wie die Ausführungsbeispiele, aber ohne Luftsauerstoffzutritt zur Referenzelektrode hergestellt sind, weisen die Ausführungsbeispiele einen kleinen Basislinienstrom und eine geringe Temperaturabhängigkeit des Basislinienstroms auf und sind somit besonders geeignet zur Detektion von kleinen Ammoniakgas-Konzentrationen weit unterhalb des TLV-Wertes und bei Umgebungstemperaturen weit oberhalb 25 °C.

### HINWEISNUMMERNVERZEICHNIS

- 1: Eintrittsöffnung für das Messgas
- 2: elektrolytfreier Hohlraum
- 3: poröser, Elektrolyt abweisender Elektrodenträger (PTFE-Membran)
- 3b, 3c: poröse PTFE-Membran
- 4: Katalysatorschicht der Arbeitselektrode
- 5: Katalysatorschicht der Referenzelektrode
- 6: Katalysatorschicht der Gegenelektrode
- 7: Elektrolyt
- 8: Eintrittsöffnung für Luftsauerstoff
- 9: Luftspalt
- 9a, 9b: Luftspalt zur Referenz- bzw Gegenelektrode
- 10: Gehäuse
- 10a, 10b, 10c: Gehäuseteile
- 11: PVDC-Ringscheibe
- 12: Glasfaser-Separator
- 13: Glasfaser-Docht
- 14: NBR-O-Ring
- 15: Kontaktdraht
- 16: Elektrodenstapel

## Patentansprüche

1. Ammoniaksensor auf amperometrischer Basis, der mit einem Potentiostat betrieben werden kann, mit einer als Diffusionsbarriere ausgebildeten Eintrittsöffnung für ein Messgas (1), einer Arbeitselektrode (4), Gegenelektrode (6) und Referenzelektrode (5) und einem Flüssigelektrolyt (7), der Co²⁺-Ionen und einen durch Luftsauerstoff oxidierbaren hygroskopischen Zusatz enthält,
**dadurch gekennzeichnet, dass**
ein Zutritt für Luftsauerstoff in Gasform zur Referenzelektrode (5) über einen Gasphasenpfad (9a, 9) erfolgt, der über eine als Diffusionsbarriere ausgebildete Eintrittsöffnung (8) für Luftsauerstoff am Sensorgehäuse (10) und Diffusionskanäle innerhalb des Sensorgehäuses (10) verläuft, wodurch ein Defizit von Sauerstoff, bedingt durch eine Oxidation eines hygroskopischen Zusatzes an der Referenzelektrode (5), permanent ausgleichbar ist, so dass ein durch den hygroskopischen Zusatz verursachter Basislinienstrom und dessen Temperaturabhängigkeit erheblich reduziert werden.

2. Ammoniaksensor nach Anspruch 1, **dadurch gekennzeichnet, dass** mindestens eine Diffusionsbarriere (3, 8, 9a, 9) den Zutritt von Messgas an die Referenzelektrode (5) beschränkt, aber genügend Luftsauerstoff zur Referenzelektrode (5) durchlässt.

3. Ammoniaksensor nach einem der Ansprüche 1 oder 2, **dadurch gekennzeichnet, dass** der Zutritt von Luftsauerstoff über den Messgaseinlass (1) erfolgt und dass das zur Arbeitselektrode (4) diffundierende Messgas an der Arbeitselektrode (4) wegreagiert, so dass der Zugang des Messgases zur Referenzelektrode (5) eingeschränkt wird.

4. Ammoniaksensor nach einem der Ansprüche 1 bis 3, **dadurch gekennzeichnet, dass** zwischen der Arbeitselektrode (4) und der Referenzelektrode (5) eine Gas absperrende Ringscheibe (11) liegt, so dass der Zugang des Messgases zur Referenzelektrode (5) eingeschränkt oder ganz abgesperrt wird.

5. Ammoniaksensor nach Anspruch 4, **dadurch gekennzeichnet, dass** die Gas absperrende Ringscheibe (11) aus PVDC, PC, PE und /oder PP besteht.

6. Ammoniaksensor nach einem der Ansprüche 1 bis 5, **dadurch gekennzeichnet, dass** mindestens die Arbeitselektrode (4) und die Referenzelektrode (5) Gasdiffusionselektroden sind.

7. Ammoniaksensor nach einem der Ansprüche 1 bis 6, **dadurch gekennzeichnet, dass** mindestens die Arbeitselektrode (4) einen Katalysator der Platinmetallgruppe, insbesondere Ruthenium, enthält.

8. Ammoniaksensor nach Anspruch 7, **dadurch gekennzeichnet, dass** mindestens die Arbeitselektrode (4) und Referenzelektrode (5) aus einem porösen PTFE-Träger bestehen, der mit einer Katalysatorschicht, die feinverteilte Ruthenium- und PTFE-Teilchen enthält, versehen ist.

9. Ammoniaksensor nach einem der Ansprüche 1 bis 8, **dadurch gekennzeichnet, dass** der hygroskopische Zusatz einen Alkohol, insbesondere Ethylenglykol und/ oder Glyzerin enthält.

10. Ammoniaksensor nach einem der Ansprüche 1 bis 9, **dadurch gekennzeichnet, dass** der Elektrolyt (7) zusätzliche, durch Luftsauerstoff nicht oxidierbare hygroskopische Bestandteile enthält.

11. Ammoniaksensor nach einem der Ansprüche 1 bis 10, **dadurch gekennzeichnet, dass** der Elektrolyt (7) ein CoSO₄-Salz enthält.

12. Ammoniaksensor nach einem der Ansprüche 1 bis 11, **dadurch gekennzeichnet, dass** zusätzliche Hilfselektroden enthalten sind.

## Claims

1. An ammonia sensor based on amperometry, which can be operated with a potentiostat, comprising an inlet opening for a measuring gas (1) which is arranged as a diffusion barrier, a working electrode (4), a counter-electrode (6) and a reference electrode (5), and a fluid electrolyte (7) which contains Co²⁺ ions and a hygroscopic additive that can be oxidized by atmospheric oxygen, **characterized in that** access for atmospheric oxygen in gas form to the reference electrode (5) occurs via a gas phase path (9a, 9) which extends via an inlet opening (8) for atmospheric oxygen arranged as a diffusion barrier on the sensor housing (10) and diffusion channels within the sensor housing (10), by means of which a deficit of oxygen caused by oxidation of a hygroscopic additive on the reference electrode (5) can be balanced permanently, so that a baseline current caused by the hygroscopic additive and its temperature dependence can be reduced substantially.

2. An ammonia sensor according to claim 1, **characterized in that** at least one diffusion barrier (3, 8, 9a, 9) limits the access of measuring gas to the reference electrode (5), but still permits the passage of sufficient atmospheric oxygen to the reference electrode (5).

3. An ammonia sensor according to claim 1 or 2, **characterized in that** the access of atmospheric oxygen occurs via the measuring gas inlet (1), and that the measuring gas diffusing to the working electrode (4) will react away on the working electrode (4), so that the access of the measuring gas to the reference electrode (5) will be limited.

4. An ammonia sensor according to one of the claims 1 to 3, **characterized in that** a gas-blocking ring disk (11) is disposed between the working electrode (4) and the reference electrode (5), so that the access of the measuring gas to the reference electrode (5) will be limited or blocked entirely.

5. An ammonia sensor according to claim 4, **characterized in that** the gas-blocking ring disk (11) consists of PVDC, PC, PE and/or PP.

6. An ammonia sensor according to one of the claims 1 to 5, **characterized in that** at least the working electrode (4) and the reference electrode (5) are gas-diffusion electrodes.

7. An ammonia sensor according to one of the claims 1 to 6, **characterized in that** at least the working electrode (4) contains a catalyst of the platinum metal group, especially ruthenium.

8. An ammonia sensor according to claim 7, **characterized in that** at least the working electrode (4) and the reference electrode (5) consist of a porous PTFE carrier which is provided with a catalytic layer containing finely distributed ruthenium and PTFE particles.

9. An ammonia sensor according to one of the claims 1 to 8, **characterized in that** the hygroscopic additive contains an alcohol, especially ethylene glycol and/or glycerine.

10. An ammonia sensor according to one of the claims 1 to 9, **characterized in that**
the electrolyte (7) contains additional hygroscopic components which cannot be oxidized by atmospheric oxygen.

11. An ammonia sensor according to one of the claims 1 to 10, **characterized in that**
the electrolyte (7) contains a CoSO₄ salt.

12. An ammonia sensor according to one of the claims 1 to 11, **characterized in that**
additional auxiliary electrodes are contained.

## Revendications

1. Détecteur d'ammoniac de type ampérométrique qui peut fonctionner avec un potentiostat, avec une ouverture d'entrée pour un gaz à mesurer (1) configurée comme une barrière de diffusion, une électrode de travail (4), une contre-électrode (6) et une électrode de référence (5) et un électrolyte liquide (7) qui contient des ions de CO²⁺ et un additif hygroscopique oxydable par l'oxygène de l'air,
**caractérisé en ce**
**qu'**une entrée pour l'oxygène de l'air sous forme de gaz vers l'électrode de référence (5) se fait par une voie de phase gazeuse (9a, 9) qui passe par une ouverture d'entrée (8) pour l'oxygène de l'air qui est configurée comme une barrière de diffusion sur le boîtier du détecteur (10) et par des canaux de diffusion à l'intérieur du boîtier du détecteur (10), ce qui fait qu'un déficit d'oxygène, dû à une oxydation d'un additif hygroscopique sur l'électrode de référence (5), peut être compensé de manière permanente si bien qu'un courant de ligne de base causé par l'additif hygroscopique et sa dépendance de la température sont considérablement réduits.

2. Détecteur d'ammoniac selon la revendication 1, **caractérisé en ce qu'**au moins une barrière de diffusion (3, 8, 9a, 9) limite l'accès de gaz à mesurer sur l'électrode de référence (5) mais laisse passer suffisamment d'oxygène de l'air vers l'électrode de référence (5).

3. Détecteur d'ammoniac selon l'une des revendications 1 ou 2, **caractérisé en ce que** l'entrée d'oxygène de l'air se fait par l'entrée de gaz à mesurer (1) et que le gaz à mesurer qui se diffuse vers l'électrode de travail (4) est résorbé au niveau de l'électrode de travail (4) si bien que l'accès du gaz à mesurer vers l'électrode de référence (5) est limité.

4. Détecteur d'ammoniac selon l'une des revendications 1 ou 3, **caractérisé en ce qu'**un disque annulaire (11) qui bloque le gaz se trouve entre l'électrode de travail (4) et l'électrode de référence (5) si bien que l'accès du gaz à mesurer vers l'électrode de référence (5) est limité ou totalement bloqué.

5. Détecteur d'ammoniac selon la revendication 4, **caractérisé en ce que** le disque annulaire (11) qui bloque le gaz est en PVDC, PC, PE et/ou en PP.

6. Détecteur d'ammoniac selon l'une des revendications 1 à 5, **caractérisé en ce qu'**au moins l'électrode de travail (4) et l'électrode de référence (5) sont des électrodes de diffusion de gaz.

7. Détecteur d'ammoniac selon l'une des revendications 1 à 6, **caractérisé en ce qu'**au moins l'électrode de travail (4) contient un catalyseur du groupe du métal platine, en particulier du ruthénium.

8. Détecteur d'ammoniac selon la revendication 7, **caractérisé en ce qu'**au moins l'électrode de travail (4) et l'électrode de référence (5) sont en un support poreux en PTFE qui est pourvu d'une couche de catalyseur qui contient des particules finement réparties de ruthénium et de PTFE.

9. Détecteur d'ammoniac selon l'une des revendications 1 à 8, **caractérisé en ce que** l'additif hygroscopique contient un alcool, en particulier de l'éthylène glycol et/ou de la glycérine.

10. Détecteur d'ammoniac selon l'une des revendications 1 à 9, **caractérisé en ce que** l'électrolyte (7) contient des composants hygroscopiques supplémentaires non oxydables par l'oxygène de l'air.

11. Détecteur d'ammoniac selon l'une des revendications 1 à 10, **caractérisé en ce que** l'électrolyte (7) contient un sel de CoSO₄.

12. Détecteur d'ammoniac selon l'une des revendications 1 à 11, **caractérisé en ce que** des électrodes auxiliaires supplémentaires sont contenues.
